# EUROPEAN PATENT SPECIFICATION

(11) **EP 1 264 585 B1**
(45) Date of publication and mention of the grant of the patent: **08.12.2004**
(21) Application number: 02100670.5
(22) Date of filing: 05.06.2002
(51) Int. Cl.: A61F 7/02

(54) **Steam evolving device**
Dampf erzeugendes Gerät
Outil produisant de la vapeur

(30) Priority: 05.06.2001 JP 2001169041
(43) Date of publication of application: 11.12.2002
(73) Proprietor: Ohshin Pharmaceutical Co. Ltd., Takefu-shi, Fukui 915-0052 (JP)
(72) Inventor: Ota, Keizo, Fukui, 915-0052 (JP); Watanabe, Tetsuhiro, Fukui, 915-0052 (JP)
(74) Representative: Weihs, Bruno

(56) References cited:
- EP-A- 0 342 927
- WO-A-99/60313
- DE-U- 29 710 493
- GB-A- 2 303 208
- US-A- 3 587 578
- US-A- 5 445 607
- US-A- 5 597 577
- US-A- 5 879 378
- US-A- 6 099 556

## Description

### Field of Invention

This invention relates to a device that can evolve steam, namely, a steam emitter. This device is used to apply to a part of an object, e.g., human body, and to give steam to that part. By the steam, the device exhibits steaming, moisturing, and moisture retaining effects at the part to which the device is applied. By the effects as a warm poultice together with the steaming effect, the device promotes blood circulation and improves metabolism. Further, the device improves pharmacological effects of percutaneous or endermic absorptive medicines and beautification effects of cosmetics, because it is useful to promote percutaneous or endermic absorption of the medicines and cosmetics.

Also, the device can cool a part of an object, e.g., human body, to which the device is applied. By the effects as a cool poultice, the device is useful to relieve inflammation, to give astringency to skin, and to give refreshing feeling.

### Description of Related Art

Skin troubles such as chronic itch, dry skin, skin roughness, chapped lip, lip roughness, chapped nasal cavity, roughness of nasal cavity, and fine lines, and asthenopia such as eye strain and dry eye occur due to, mainly, dryness of skin, mucous membrane, and eyeballs. Therefore, to prevent these skin troubles and asthenopia, it is best to avoid dryness by supplying a suitable amount of water or moisture to the skin and eyeballs and giving moisture to them.

Conventionally, as means for supplying moisture to prevent dryness of skin, eyeballs, and the like, uses of a hot towel that was warmed with hot water, a steaming hot towel, a humidifier comprising an electric heater or supersonic wave generator, and a device for facial beautification by steam have been adopted.

However, when a hot towel that was warmed with hot water or a steaming hot towel is used, it is difficult to control the temperature of the towel. Namely, if the towel is too hot, the user may be scalded itself with the towel. Also, water that seeps away from the towel may stain clothes. Thus, using those towels lacks safety and convenience. Further, the treatment for warming a towel with a hot water or by a heater is complicated. The temperatures of those towels lower in a short time of period and as a result comfortableness is reduced.

While, if a user wants to use a humidifier comprising an electric heater or supersonic wave generator or a device for facial beautification by steam, he must arrange a special device, i.e., the humidifier or device for facial beautification. The device cannot be used at an optional place at any time. Therefore, the use of the device lacks convenience and simplicity.

Japanese Patent Early-publication No. Hei. 11-342147 (hereafter "D-147") discloses a steam generator, which had been invented to solve the above-mentioned problems. This steam generator can conveniently supply warmth and moisture to any part of skin or mucous membrane of human body at any time.

This steam generator has a steam-generating section using a chemical energy and is applied to skin or mucous membrane. The component part that faces skin or a mucous membrane is made of a moisture-permeable sheet. The temperature of steam that is evolved from the surface of the sheet is controlled to be 50°C or less. D-147 discloses, as examples of the chemical energy, heat of neutralization, heat of hydration of inorganic salts (e.g., calcium chloride, magnesium chloride, calcium oxide, magnesium oxide, and zolites), and heat generated by oxidation of metal powders. D-147 discloses, as a specific example, a steam-generating section comprising a heating section where heat of neutralization or heat of hydration is generated and an evaporating section from which steam is evolved by the action of the generated heat. D-147 discloses that heat-generatable substances or compositions that can also generate steam are preferably used in the steam-generating section. This is because there is no need to make the evaporating section in addition to the heating section. D-147 discloses, as an example of the heat-generatable substances or compositions that can also generate steam, a composition comprising a metal powder, a salt, and water. This composition evolves steam by oxidation reaction.

This steam generator that D-147 discloses has some problems. Namely, first, the user cannot control the temperature of heat, because in that steam generator the temperature is previously set. Second, in the case where cooling is needed, e.g., where there is an inflammation, this steam generator cannot be used. Third, there is a problem that results from the difficulty of the control of the heat of neutralization or heat of hydration of an inorganic compound, although D-147 says that the temperature of generating steam can be controlled when the steam generator is used. If the temperature becomes too high, the part to which the steam generator is applied may be burned or inflamed. This third problem is important because it relates to safety.

Furthermore, when the steam generator is not used, no chemical reaction should occur. Therefore, the steam generator should be enclosed in an air-proofed bag, namely, sealed. Once the bag is opened, the chemical reaction cannot be stopped and the chemical energy is used up. Namely, the fourth problem is that the steam generator cannot be repeatedly used and therefore is uneconomical.

US patent No. 5,597,577 describes a device comprising a water-permeable envelope and a gel forming material that is contained in the envelope. By immersing the device in water, water enters through the envelope. Then, the gel-forming material becomes a gel with water. The envelope maintains a wicking action which slowly draws water from the interstices of the gel material to the surface of the textile envelope. The water at the textile/air interface will then evaporate.

### Summary of Invention

The present inventors had extremely studied to solve the above-discussed problems. As a result, they have accomplished the present invention.

Namely, the present invention is a device that can evolve steam according to claim 1. Preferred embodiments are described in the appended dependent claims.

### Brief Description of Drawing

Figure 1 is a sectional view of the first embodiment of the device according to the present invention.
Figure 2 is a sectional view of the second embodiment of the device according to the present invention.
Figure 3 is a sectional view of the third embodiment of the device according to the present invention.
Figure 4 is a sectional view of the fourth embodiment of the device according to the present invention.
Figure 5 is a perspective view of the fourth embodiment of the device according to the present invention.

### Detailed Description of Invention

Hereafter, the present invention is particularly explained.

First, we explain the water-bearing gel of the device according to the present invention.

The water-bearing gel comprises, as essential components, a water-absorbable polymer and water. The kind of gel is not limited as long as it comprises a water-absorbable polymer and water. Known water-bearing gels, for example, those that have been used in known sheets for cooling down and in gel pads for warming up, may be used. Water-absorbable polymers that can be used are polymeric materials that can readily absorb a large quantity of water and become gel, e.g., known hydrophilic polymers. Specific examples of water-absorbable polymers include, but are not limited to, synthetic hydrophilic polymers such as poly(vinyl alcohol), poly(vinyl pyrrolidone), poly(acrylic acid), polyacrylate, and maleic-anhydride-type copolymer; natural hydrophilic polymers such as dextran, dextrin, pulullan, and gelatin; and semisynthetic hydrophilic polymers such as methylcellulose, ethylcellulose, and carboxy methylcellulose. In the present invention, one or more water-absorbable polymers may be used to make the water-bearing gel.

In the present invention, cross-linked water-absorbable polymers may also be used. By treating a polymer with a crosslinking agent, the viscosity of the water-bearing gel can be suitably controlled. If the gel has a suitable viscosity, its shape can be readily maintained. Examples of crosslinking agents include, but not limited thereto, acetaldehyde, glutaraldehyde, glyoxal, dialdehyde starch, and dimethylketone. Combinations of polymers and crosslinking agents are known in this technical field. Treating methods are also known.

To enhance hydrophilicity, these polymers may be treated with a surfactant, or, the water-bearing gel may comprise a surfactant. Surfactants are also useful to improve dispersion ability of materials and substances. Examples of surfactants that can be used include, but are not limited to, anionic surfactants such as soaps of higher fatty acids, salts of alkyl sulfates, salts of poly(oxyethylene)alkylether sulfates, salts of acyl N-methyltaurines, salts of alkyletherphosphate, and salts of N-acylaminoacid; cationic surfactants such as alkyl trimethyl ammonium chlorides, dialkyl dimethyl ammonium chlorides, and benzalkonium chloride; amphoteric surfactants such as betaine alkyldimethylaminoacetates, betaine alkylamidodimethylaminoacetates, and 2-alkyl-N-hydroxyimidazolinium betaines; nonionic surfactants such as polyethyleneglycol type surfactants, polyhydric alcohol ester type surfactants, and ethylene oxide-propylene oxide block copolymer type surfactants; polymeric surfactants such as solbitan monolaurate; and natural surfactants.

The device of the present invention may be cooled. Therefore, to prevent that the gel becomes hard or rigid, the water-bearing gel preferably comprises an antifreezing agent. Examples of antifreezing agents include, but not limited thereto, mixtures of inorganic compounds (e.g., calcium chloride), and organic compounds such as ethylene glycol, glycerol, propylene glycol, poly(ethylene glycol), diethylene glycol, diethylene glycol monoalkyl (e.g., C₁₋₃)ethers, diethylene glycol dialkyl(e.g., C₁₋₃)ethers, triethylene glycol monoalkyl(e.g., C₁₋₃)ethers, triethylene glycol dialkyl(e.g., C₁₋₃)ethers, and glycolether alkylacetates represented by the formula: R¹-(O-R²)ₙ-O-CO-R³ (wherein R¹ and R³ are each an alkyl group having 1 to 3 carbon atoms, R² is methylene or ethylene group, and n is an integer of 1 to 5, especially 1 to 3).

The water-bearing gel may also contain additives. Additives are useful, for example, to control the viscosity of the water-bearing gel or to maintain the shape of it. Examples of additives to be used include, but not limited thereto, wood powder, pulp, pulp powder, kaolin, bentonite, vermiculite, silica powder, clay, pearlite, zeolite, zinc white, titanium oxide, talc, aluminum chloride, aluminum sulfate, aluminum nitrate, aluminum hydroxide, potassium aluminum sulfate, organic spherical powders such as spherical cellulose, and polymers such as polybutene and polyisobutylene.

The water-bearing gel may contain, at need, dyes and pigments to color the gel.

The water content of the gel according to the present invention is suitably decided depending on the kind of the water-absorbable polymer and that of the additive, and is not limited. However, the water content is preferably 50 to 97.5 % by weight, more preferably 60 to 95 % by weight of the entire weight of the gel. Especially in these ranges, the gel can bear or hold the water and can give suitable effects by evolving steam.

The thickness of the gel is, but is not limited to, generally 5 to 5000 µm, preferably 20 to 2500 µm, more preferably 35 to 1500 µm. Especially in these ranges, the effects resulting from evolving steam and warming up, or the effects resulting from cooling down last for a long time of period, the feeling of the device becomes comfortable, and the production cost is economical or reasonable.

The gel lies between a moisture-permeable base material and a covering material.

The base material is moisture-permeable. Therefore, the water or moisture that is born or held in the gel can pass through the base material. Thus, the device of the present invention can suitably supply steam to a part of an object to which the device is applied.

The moisture permeability of the base material affects the steaming effects of the device of the present invention. Therefore, it is preferable to use a base material having a moisture permeability of 2,000 to 6,000 g/m²· 24 hours, especially 2,500 to 4,500 g/m²·24 hours by the Lyssy method.

The Lyssy method is compliant to industry standards of many countries. In, e.g., JIS Z 0208 and JIS K 7129, the measurement by the Lyssy method is conducted at 40°C under a difference of relative humidities of 90 %. More particularly, a sample to be measured is inserted to the interface of an underneath chamber that lies under a condition of relative humidity of 100 % and an upper chamber comprising a high-sensitive humidity sensor. The relative humidity of the upper chamber is maintained at 10 %. Thus, the difference of the relative humidities is 90 % (i.e., 100-10). Then, the time (seconds) to be required for increasing the relative humidity of the upper chamber from about 9 % to about 11 % is measured. By using a standard sample, of which the moisture permeability is known, the time is measured in the same way under the same condition. Comparing the data of the sample to be measured with that of the standard sample, the moisture permeability of the sample to be measured is decided.

The base material preferably comprises at least one of a cloth material made of a natural and/or artificial fiber, a polymeric film, and a polymeric sheet. The base material may be a laminate that was produced by laminating at least two of cloth materials, polymeric films, and polymeric sheets. Laminates have improved strengths. By producing a laminate, the moisture permeability of the base material can be readily controlled. A laminate that was made by laminating a cloth material (to be an outside of the device of the present invention) onto a polymeric film or sheet (to face the water-bearing gel) is preferable. When this laminate is used, the feeling of the device against a user's skin is comfortable. Also, water difficultly leaks out from the gel and therefore it is prevented to get clothes dirty.

When a cloth material that was made of a hydrophilic fiber is used, the cloth material gets wet and the latent heat of evaporation of the water that was impregnated in the cloth material cools down the skin. Therefore, a cloth material that was made of a hydrophobic fiber is preferred.

Examples of cloth materials include, but not limited thereto, a cloth, a towel cloth, a blanket's cloth, a quilt, a knitted fabric, an unwoven fabric, and a woven fabric.

Examples of natural fibers include, but not limited thereto, plant fibers such as cotton fiber, kapok, flax yarn, ramie, hemp yarn, jute yarn, bass, Manila fiber, sisal fiber, and coir; and animal fibers such as silkworm's silk, wool (sheep), cashmere wool, camel's hair, alpaca wool, mohair, and rabbit (e.g., Angora-rabbit) hair.

Examples of artificial fibers include, but are not limited to, regenerated fibers such as rayon, spun rayon, viscose rayon, and Bemberg (trade mark); synthetic fibers such as polyamide fibers, polyester fibers (e.g., Tetron (trade mark), Terylene (trade mark), and Dacron (trade mark)), polyacrylic fibers (e.g., Orlon (trade mark), Exlan (trade mark), Bonnel (trade mark), Cashimilon (trade mark), and Kanekalon (trade mark)), poly(vinyl alcohol) fibers (e.g., Vinylon (trade mark)), polyalkyleneparaoxybenzoate fibers, polyurethane fibers (e.g., Spandex (trade mark)), poly(vinylidene chloride) fibers, poly(vinyl chloride) fibers, polyacrylonitrile fibers, polyethylene fibers, polypropylene fibers (e.g., Pylene (trade mark)), and polychlal fibers; and semisynthetic fibers such as acetate fibers.

Examples of raw materials of the polymeric film or sheet include, but are not limited to, polyurethane, polyethylene, ethylene-vinyl acetate copolymer, poly(vinyl chloride), polyamide, polyester, polyacetal, polysulfone, poly(phenylene oxide), polybutadiene, polypropylene, regenerated cellulose, polychloroprene, poly(amino acid), nitrile rubber, butyl rubber, and silicone rubber. Among films and sheets that were made of these polymers, moisture-permeable ones can be used as they are. If moisture-impermeable polymers are used, a solvent-soluble filler is mixed with a polymer, and the filler is dissolved with a solvent after a film or sheet is produced. Or, filler is mixed with a polymer and to produce a film or sheet, orientation treatment is effected. Further, holes or pores may be made by physically drilling a film or sheet.

The basic weight of the base material is, for example, 5 to 1500 g/m², generally 10 to 500 g/m², preferably 15 to 350 g/m², and more preferably 17.5 to 300 g/m². When the basic weight is within these ranges, a suitable mechanical strength can be readily obtained and the thickness can be readily uniformed. Also, a comfortable flexibility can be readily realized, and the device can readily follow the form of the part to which the device is applied.

Also, the covering material is preferably made of at least one of a cloth material made of a natural and/or artificial fiber, a polymeric film, and a polymeric sheet. Examples of cloth materials and those of raw materials of the polymeric film or sheet are the same as those that are listed for the moisture-permeable base material. However, it is preferred that the covering material is impermeable or has a low permeability. Therefore, if a material having permeability is used for the covering material, by a known method the material is generally processed to lower the permeability. The moisture permeability of the covering material is preferably 200 g/m²·24 hours or less, more preferably 100 g/m^{2.}24 hours or less. It is best that the covering material is a film or sheet that is made of a gas barrier polymer.

The basic weight of the covering material is, for example, 5 to 1500 g/m², generally 10 to 500 g/m², preferably 15 to 350 g/m², and more preferably 17.5 to 300 g/m². When the basic weight is within these rages, a suitable mechanical strength can be readily obtained and the thickness can be readily uniformed. Also, a comfortable flexibility can be readily realized, and the device can readily follow the form of the part to which the device is applied.

In one embodiment of the present invention, the water-bearing gel has an area that is somewhat smaller than those of the moisture-permeable base material and the covering material, and the base material and the covering material are adhered or fused at the peripheries of them. Namely, the gel is completely covered with the base material and the covering material. In this embodiment, water in the gel unlikely leaks from the device and thus clothes unlikely get dirty. However, of course, steam can pass through the base material.

In one embodiment, the device of the present invention further comprises a layer having tackiness ("tacky layer") on the base material in all or part of the base material, preferably all or part of the periphery of the base material. For example, the tacky layer is made on both sides of the periphery of the base material, if the device is rectangular or square. The tacky layer is used to adhere the device of the present invention to the part to which the device is applied, e.g., a body surface.

When the covering material is moisture-impermeable and when the tacky layer lies on whole area of the base material, steam is difficultly evolved from the gel. Therefore, in this case the tacky layer should be made on only part of the base material.

The tacky layer may lie on the covering material. In this embodiment, the device is adhered to, e.g., an inside of underwear, and the base material faces an object, e.g., human body.

In the present invention, for the tacky layer known adhesives can be used. Those having a strong adhesive force and oleophilicity are preferred. Examples of those adhesives include, but are not limited to, acrylic compounds such as (meth)acrylates, e.g., butyl acrylate, butyl methacrylate, pentyl acrylate, pentyl methacrylate, hexyl acrylate, and hexyl methacrylate; and copolymers of a (meth)acrylate with a functional monomer (e.g., acrylic acid, methacrylic acid, itaconic acid, maleic acid, maleic anhydride, hydroxyethyl acrylate, hydroxyethyl methacrylate, hydroxypropyl acrylate, hydroxypropyl methacrylate, acrylamide, methacrylamide, dimethylacrylamide, methylaminoethyl methacrylate, methoxyethyl acrylate, and methoxyethyl methacrylate) or a vinyl monomer (e.g., acrylonitrile, vinyl acetate, vinyl propionate, and vinyl pyrrolidone).

Examples of other adhesives include, but are not limited to, rubbery adhesive materials comprising, as a main component, a rubber (e.g., silicone rubber, polyisoprene rubber, polyisobutylene rubber, polybutadiene, stylene-butadiene-stylene block copolymer, stylene-isoprene-stylene block copolymer, acrylic rubber, and natural rubber) and vinyl-type adhesive materials comprising, as a main component, a vinyl polymer (e.g., poly(vinyl ether), poly(vinyl alcohol), and poly(vinyl acetate)).

When the device is not used, it is preferable that the surface of the tacky layer is covered with a releasable covering film or sheet. Preferable examples of releasable covering films or sheets include, but are not limited to, films and sheets that are made of polymers such as polyurethane, ethylene-vinyl acetate copolymer, poly(vinyl chloride), polybutadiene, polypropylene, regenerated cellulose, polychloroprene, poly(amino acid), nitrile rubber, butyl rubber, and silicone resin. The releasable covering film or sheet may have a patterned indented surface. In this case, the area that the releasable covering film or sheet adheres to the tacky layer becomes small, because only parts of the covering film or sheet adheres to the tacky layer. The releasable covering film or sheet may be treated with a releasing agent.

In an embodiment, only a part of the tacky layer can be used. Namely, substantial area of the tacky layer is changable. In this case the area of the part having tackiness is flexible. Therefore, depending upon the purpose of using the device or the part to which the device is applied, the adhesive strength or the extent that the device can follow the form of the part to which it is applied is changeable and thus the device is conveniently used.

Means by which the substantial area of the tacky layer changes are, for example, 1) that the tacky layer is made so that a part of it can be broken away, 2) that a covering film is attached to a part of the tacky layer, and 3) that a releasable covering film or sheet is made so that a part of it can be released.

Another embodiment of the device of the present invention further comprises an accessory for controlling moisture permeability of the base material. The accessory is a part of the base material or an independent part. The device of the present invention preferably also comprises a means for holding the accessory. The accessory for controlling moisture permeability is preferably detachable. By detaching all or part of accessories, the user can control the moisture permeability of the device and, as a result, the extent of effects resulting from steam can be controlled.

The means for holding the accessory is attached to the base material. The means is, for example, a pocket, a Velcro (trade mark) fastening, a snap fastener, or a belt.

Examples of the accessories for controlling moisture permeability include, but are not limited to, those of the cloth materials, polymeric films, and polymeric sheets that are listed for the moisture-permeable base material. The accessory is preferably thin for maintaining the feeling of the device well. Thus, films and sheets that are made of at least one of polyurethane, polyethylene, ethylene-vinyl acetate copolymer, poly(vinyl chloride), polyamide, polyester, polyacetal, polysulfone, poly(phenylene oxide), polybutadiene, polypropylene, regenerated cellulose, polychloroprene, poly(amino acid), nitrile rubber, butyl rubber, and silicone rubber are preferred.

If the moisture permeability of the accessory should be controlled, for example, a polymeric solution may be applied to the accessory, or, pores or holes are made by a known method.

The base material may support, hold, or impregnate at least one member selected from the group consisting of a percutaneous or endermic absorptive medicine, a cosmetic, and a far-infrared radiator. Also, the water-bearing gel may support, hold, or contain at least one member selected from the group consisting of a percutaneous or endermic absorptive medicine, a cosmetic, and a far-infrared radiator. By the moisture that is supplied from the gel, the stratum corneum is loosened, and therefore effective components of the medicine or cosmetic are readily absorbed through or readily kept in the stratum corneum. Thus, they can be effectively administered.

In the present invention, known percutaneous or endermic absorptive medicines can be used. Examples of those medicines include, but not limited thereto, corticosteroids, anti-inflammatory and analgesic agents, anesthetics, sedatives, tranquilizers, antihypertensive agents, antibiotics, antimicrobial agents, vitamins, antiepilepsy agents, vasodepressors for coronary blood vessels, antihistaminic agents, and antifungals. Also, odorless crystals that can sublimate, peppermint oil, eucalyptus oil, rosemary oil, and lavender oil may be added.

Those that are used as poultice, e.g., boric acid solution, physiological saline, aqueous magnesium sulfate, aqueous alcohol, flax seed oil, lime water, cod-liver oil, olive oil, aqueous acrynol (Rivanol (trade name)), potassium permanganate solution, Menta water (aqueous menthol), creosote, and mustard seed may be contained in the water-bearing gel. Or, in the form of micropowder or granulated powder, active ingredients of those poultices may be supported or held on the base material.

In the present invention known cosmetics can be used. Examples of those cosmetics include, but not limited thereto, cosmetics (e.g., barrier creams) for preventing skin roughness comprising at least one of an anti-inflammatory agent, an astringent, a refrigerant, a vitamin, a hormone drug, and antihistaminic agent; cosmetics for preventing or treating acne having an action of anti-sebum secretion or an action of flaking or dissolving keratin; cosmetics containing an animal or plant extract (e.g., aloe extract, ginseng extract, or licorice extract); and cosmetics containing a nutritional supplement (e.g., an amino acid).

The base material, water-bearing gel, or both can support or hold a far-infrared radiator. The radiator is dispersed in the base material or water-bearing gel.

Generally, the term "far-infrared" means an electromagnetic wave having a wavelength of about 25 - 100 µm. However, in this specification, about the term "far-infrared," its wavelength is not stringently restricted. If wavelength is close to the range of about 25 - 100 µm, the electromagnetic wave is included in the definition of the "far-infrared." The term "far-infrared radiator" means substances having a function of radiating far-infrared.

In the present invention known far-infrared radiators can be used. Examples those radiators include, bur not limited thereto, inorganic solids of nonmetals having a function of radiating far-infrared, namely, ceramics that can radiate far-infrared. Specifically, those ceramics are oxides, carbides, nitrides, and carbonates of metals. Examples of the metals that constitute the ceramics include, bur not limited thereto, Al, Be, V, Fe, Y, Co, Cu, Ni, Si, Sn, Ti, Cr, Ce, Zr, Ca, Ta, and Nb, In the present invention ceramics in the form of a film or sheet, powder, or granule may be preferably used.

Before applying to, e.g., human body, the device of the present invention is warmed by microwave in an electronic oven. Or, it is cooled in a refrigerator or freezer.

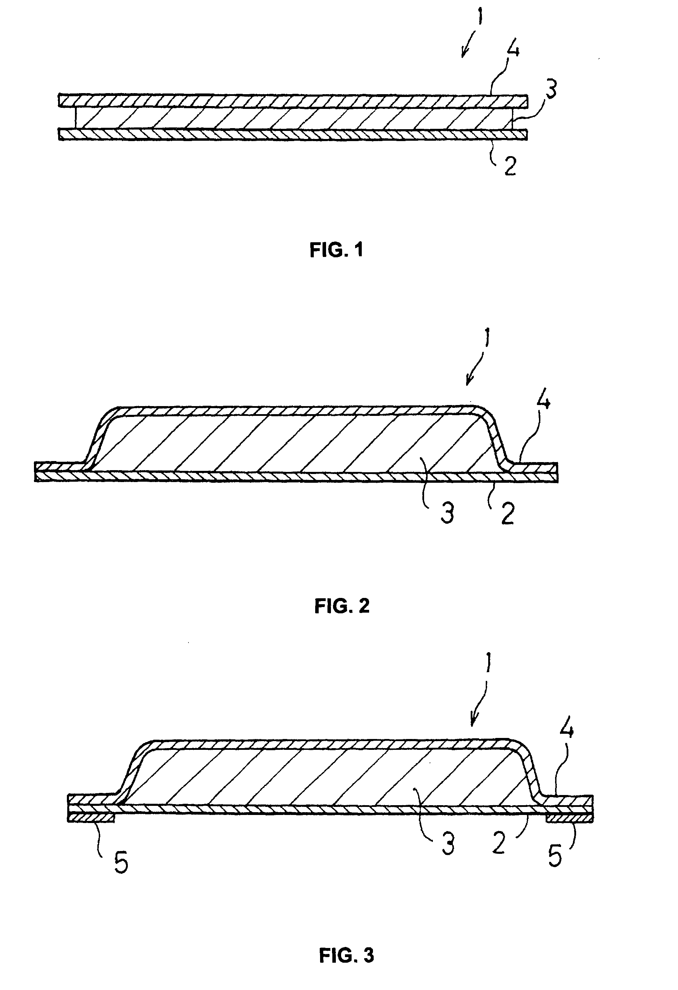

Next, with referring to figures 1-5, the constitution of the device of the present invention is explained.

Figure 1 is a sectional view of the first embodiment of the device 1 according to the present invention. The device 1 comprises a moisture-permeable base material 2, a water-bearing gel 3, and a covering material 4. The base material 2 and the covering material 4 are stuck on the water-bearing gel 3 by the tackiness of the gel 3.

Figure 2 is a sectional view of the second embodiment of the device according to the present invention. In this embodiment, the periphery of the base material 2 is adhered to or fused into the covering material 4. Namely, the gel 3 is entirely sealed in.

Figure 3 is a sectional view of the third embodiment of the device according to the present invention. This device 1 has layers 5, 5 having tackiness on the base material 2 in both sides of the periphery of the base material 2. By these layers 5, 5, the device 1 is adhered to an object, e.g., human body.

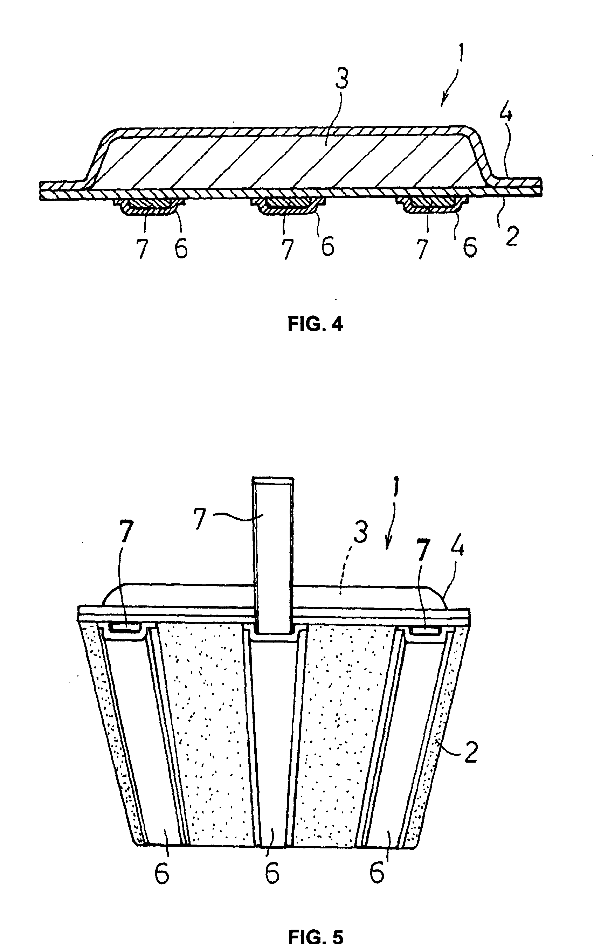

Figure 4 is a sectional view of the fourth embodiment of the device according to the present invention. Figure 5 is a perspective view of the fourth embodiment of the device according to the present invention. This device 1 has means 6, 6, 6 for holding accessories 7, 7, 7. The accessories 7, 7, 7 are used to control the moisture permeability of the base material 2. The accessories 7, 7, 7 are each detachable.

The device of the present invention can be warmed and evolve steam. Thus, the device can warm up and give steam to a part of human body where the device is applied. Therefore, it exhibits steaming, moisturing, and moisture retaining functions together with functions as a warm poultice. By these functions, blood circulation is promoted and metabolism is improved. Further, pharmacological effects of percutaneous or endermic absorptive medicines and beautification effects of cosmetics are improved, because the above functions are useful to promote percutaneous or endermic absorption of the medicines and cosmetics. The steaming, moisturing, and moisture retaining functions are obtained, because through the base material the moisture that is held in a gel is supplied to the part of human body where the device is applied.

Also, the device can be cooled. Thus, it can cool down a part of human body where the device is applied. By this function as a cool poultice, inflammation is relieved, and an astringent effect to skin and refreshing feeling are obtained.

The device of the present invention can be repeatedly warmed by using, e.g., an electronic oven and can be repeatedly cooled by using, e.g., a refrigerator or freezer. Therefore, it is economical.

The device of the present invention can be produced to have a thin body. Also, due to the use of gel, a comfortable flexibility is realized. Namely, the device can readily follow the form of the part where the device is applied and can exhibit comfortable feeling to that part.

Before applying the device to the part where the device is applied, the user can check the temperature of the device. Thus, a burn or excess cooling can be prevented. Further, if a device in which the base material comprises two or more materials (i.e., the base material is a laminate of two or more layers) is used, the burn (including a burn resulting from contacting with a low-temperature material in a longer time of period) or excess cooling can be prevented more sure.

Further in a embodiment that the base material or the water-bearing gel supports, holds, impregnates, or contains a percutaneous or endermic absorptive medicine or a cosmetic, the effective components of the medicine or cosmetic can be readily absorbed through or readily kept in stratum corneum, can be effectively administered, and thus can exhibit their functions more effectively. This is because the stratum corneum is loosened by steam.

### Examples

### 1. Explanation of the materials used

### (1) Water-bearing gel

By a known method, a water-bearing gel was produced by using the components listed in Table 1.

**Table 1**

| Name of Components (Trade Name) | Amount (Unit: % by weight) |
|---|---|
| Poly(vinyl alcohol) (Poval LA-18) | 1 |
| Acrylic acid starch (Sanwet) | 0.8 |
| Carboxymethylcellulose (CMC) | 0.5 |
| Eucalyptus oil | 0.025 |
| Rosemary oil | 0.015 |
| Tartaric acid | 0.36 |
| Butyl parahydroxybenzoate (Paraben Butyl) | 0.05 |
| Methyl parahydroxybenzoate (Paraben Mehtyl) | 0.05 |
| Propylene glycol | 0.1 |
| Kaolin | 7 |
| Sodium polyacrylate (Aronbis AH-105) | 3.8 |
| Dried aluminum hydroxide gel | 0.06 |
| Magnesium metasilicate aluminate (Neusilin) | 0.03 |
| Sodium edetate (EDTA) | 0.005 |
| Conc. glycerol | 7.6 |
| Water | 78.605 |
| Total | 100 |

### (2) Base material

A three-layered material (70 mm × 140 mm) was used. The material was produced by laminating a porous film made of polyethylene (thickness: 40 µm) on an unwoven fabric made of polyester (basic weight: 30g/m²), and then laminating an unwoven fabric made of polypropylene (basic weight: 30g/m²) on the porous film.

The permeability of the base material was 3,860 g/m^{2.} 24 hours by the Lyssy method.

### (3) Covering material

A three-layered material (70 mm × 140 mm) was used. The material was produced by laminating a gas barrier film made of polyethylene (thickness: 20 µm) on an unwoven fabric made of polyester (basic weight: 30g/m²), and then laminating an unwoven fabric made of polypropylene (basic weight: 30g/m²) on the gas barrier film.

### 2. Production of the devices of the present invention Devices 1, 2, and 3 were produced by the following methods.

### (1) Device 1 (see Figure 1)

On the unwoven fabric made of polypropylene of the base material, the composition (water-bearing gel) shown in Table 1 was applied to be a size of 60 mm × 130 mm and a basic weight of 1800 g/m². Then, the covering material was laminated on the gel so that the unwoven fabric made of polypropylene faces the gel.

### (2) Device 2 (see Figure 2)

The procedure written in item (1) was repeated. Thereafter, the periphery of the base material was heat-sealed with that of the covering material. Thus, the gel was entirely sealed in.

### (3) Device 3 (see Figure 3)

The procedure written in item (2) was repeated. Thereafter, on the base material layers having tackiness were made in both sides of the periphery of the base material in a width of 15 mm. By using an adhesive comprising a stylene-isoprene-stylene block copolymer as the main ingredient, the layers were produced.

### 3. Test

Ten patients who had noticed symptoms of asthenopia used Devices 1, 2, and 3 by putting each of them on eyelids for five minutes. Before the test, Devices 1, 2, and 3 were warmed by using an electronic oven of 500 W (watt) for thirty seconds, or cooled in a refrigerator having a temperature of 4°C for one hour.

The patients were women forty-two to fifty-five years old.

### 4. Result

### (1) Warming test

For each of Devices 1, 2, and 3, eight or nine patients said that the symptoms of asthenopia were remarkably relieved.

One or two patients said that the symptoms of asthenopia were relieved to some extent.

No patient said that there was no effect.

Because Device 3 could be fixed on eyelids, was not shifted, and satisfactorily adhered to eyelids, nine patients said that the symptoms of asthenopia were remarkably relieved.

### (2) Cooling test

For each of Devices 1, 2, and 3, seven or eight patients said that the symptoms of asthenopia were remarkably relieved.

Two or three patients said that the symptoms of asthenopia were relieved to some extent.

No patient said that there was no effect.

The present invention is defined or limited only by the claims.

## Claims

1. A steam evolving device (1) **characterized in that** the device comprises a moisture-permeable base material (2) that comprises a moisture-permeable polymeric film or sheet, a water-bearing gel (3) that comprises a water-absorbable polymer and water, and a covering material (4), which are laminated in this order, wherein the base material (2) and the covering material (4) are adhered or fused at the peripheries of them, **characterized in that** the moisture permeabilities of the base material and the covering material are controlled within the range of, by the Lyssy method, 2500 to 4500 g/m^{2.}24 hours and 200 g/m^{2.}24 hours or less, respectively.

2. The device according to claim 1, wherein the base material (2) further comprises a cloth material made of a natural and/or artificial fiber on the outer surface of the moisture-permeable polymeric film or sheet.

3. The device according to claim 2, wherein the cloth material is selected from the group consisting of a cloth, a towel cloth, a blanket's cloth, a quilt, a knitted fabric, an unwoven fabric, and a woven fabric.

4. The device according to claim 1, wherein the polymeric film or sheet is made of at least one member selected from the group consisting of polyurethane, polyethylene, ethylene-vinyl acetate copolymer, poly(vinyl chloride), polyamide, polyester, polyacetal, polysulfone, poly(phenylene oxide), polybutadiene, polypropylene, regenerated cellulose, polychloroprene, poly(amino acid), nitrile rubber, butyl rubber, and silicone rubber.

5. The device according to claim 1 or 2, wherein the base material (2) further comprises, for controlling the moisture permeability of the base material (2), a film or sheet (7) that is made of at least one member selected from the group consisting of polyurethane, polyethylene, ethylene-vinyl acetate copolymer, poly(vinyl chloride), polyamide, polyester, polyacetal, polysulfone, poly(phenylene oxide), polybutadiene, polypropylene, regenerated cellulose, polychloroprene, poly(amino acid), nitrile rubber, butyl rubber, and silicone rubber on the outer surface of the moisture permeable polymeric film or sheet or the cloth material.

6. The device according to claim 5, which further comprises a means (6) for holding the film or sheet (7) for controlling the moisture permeability of the base material (2) on the outer surface of the base material (2), wherein the means (6) is selected from the group consisting of a pocket, a Velcro (trademark) fastening, a snap fastener, and a belt.

7. The device according to claim 1, which further comprises a layer (5) having tackiness on the base material (2) in all or part of the periphery of the base material (2).

8. The device according to claim 7, wherein the effective area of the layer (5) having tackiness is changeable.

9. The device according to claim 1, wherein the base material (2) supports, holds, or impregnates at least one member selected from the group consisting of a percutaneous or endermic absorptive medicine, a cosmetic, and a far-infrared radiator.

10. The device according to claim 1, wherein the water-bearing gel (3) supports, holds, or contains at least one member selected from the group consisting of a percutaneous or endermic absorptive medicine, a cosmetic, and a far-infrared radiator.

## Patentansprüche

1. Dampfbildende Vorrichtung (1), **dadurch gekennzeichnet, dass** die Vorrichtung ein feuchtigkeitsdurchlässiges Grundmaterial (2) mit einem feuchtigkeitsdurchlässigen Polymerfilm oder einer Polymerfolie, ein wassertragendes Gel (3) mit einem in Wasser absorbierbaren Polymer und Wasser und ein in dieser Reihenfolge laminiertes Abdeckmaterial (4) umfasst, wobei das Grundmaterial (2) und das Abdeckmaterial (4) am Umfang aneinander festgeklebt oder verschmolzen sind, **dadurch gekennzeichnet, dass** die Feuchtigkeitsdurchlässigkeiten des Grundmaterials und des Abdeckmaterials nach der Methode von Lyssy im Bereich von 2500 bis 4500 g/m² x 24 Stunden bzw. 200 g/m² x 24 Stunden oder weniger kontrolliert werden.

2. Vorrichtung nach Anspruch 1, worin das Grundmaterial (2) ferner ein Tuchmaterial aus einer natürlichen und/oder synthetischen Faser auf der Außenseite des feuchtigkeitsdurchlässigen Films oder der Folie umfasst.

3. Vorrichtung nach Anspruch 2, worin das Tuchmaterial aus der aus Tuch, Handtuchstoff, Deckenstoff, Steppstoff, Gewirke, Vlies und Gewebe bestehenden Gruppe ausgewählt ist.

4. Vorrichtung nach Anspruch 1, worin der Polymerfilm bzw. die Polymerfolie aus mindestens einem aus der aus Polyurethan, Polyethylen, Ethylenvinylacetat-Copolymer, Poly(vinylchlorid), Polyamid, Polyester, Polyacetal, Polysulfon, Poly(phenylenoxid), Polybutadien, Polypropylen, regenerierte Cellulose, Polychloropren, Poly(aminosäure), Nitrilkautschuk, Butylkautschuk und Silikonkautschuk bestehenden Gruppe ausgewählten Mitglied besteht.

5. Vorrichtung nach Anspruch 1 oder 2, worin das Grundmaterial (2) zur Kontrolle der Feuchtigkeitsdurchlässigkeit des Grundmaterials (2) ferner einen Film oder eine Folie (7) umfasst, der bzw. die aus mindestens einem aus der aus Polyurethan, Polyethylen, Ethylenvinylacetat-Copolymer, Poly(vinylchlorid), Polyamid, Polyester, Polyacetal, Polysulfon, Poly(phenylenoxid), Polybutadien, Polypropylen, regenerierte Cellulose, Polychloropren, Poly(aminosäure), Nitrilkautschuk, Butylkautschuk und Silikonkautschuk bestehenden Gruppe ausgewählten Mitglied auf der Außenseite des feuchtigkeitsdurchlässigen Films oder der Folie oder des Tuchmaterials besteht.

6. Vorrichtung nach Anspruch 5, die ferner ein Mittel (6) zum Halten des Films oder der Folie (7) zur Kontrolle der Feuchtigkeitsdurchlässigkeit des Grundmaterials (2) auf der Außenseite des Grundmaterials (2) umfasst, worin das Mittel (6) aus der aus einer Tasche, einem Klettverschluss (Velcro-Verschluss, Warenzeichen), einem Schnappverschluss und einem Gürtel bestehenden Gruppe ausgewählt ist.

7. Vorrichtung nach Anspruch 1, die ferner eine Schicht (5) mit Klebrigkeit auf dem Grundmaterial (2) in der gesamten oder einem Teil der Peripherie des Grundmaterials (2) aufweist.

8. Vorrichtung nach Anspruch 7, worin der wirksame Bereich der Schicht (5) mit Klebrigkeit veränderlich ist.

9. Vorrichtung nach Anspruch 1, worin das Grundmaterial (2) mindestens ein aus der aus einem perkutanen oder intrakutanen absorptionsfähigen Medikament, einem kosmetischen Mittel und einem Ferninfrarotstrahler bestehenden Gruppe ausgewähltes Mitglied stützt, hält oder imprägniert.

10. Vorrichtung nach Anspruch 1, worin das wassertragende Gel (3) mindestens ein aus der aus einem perkutanen oder intrakutanen absorptionsfähigen Medikament, einem kosmetischen Mittel und einem Ferninfrarotstrahler bestehenden Gruppe ausgewähltes Mitglied stützt, hält oder enthält.

## Revendications

1. Dispositif (1) dégageant de la vapeur **caractérisé en ce que** le dispositif comprend un matériau de base (2) perméable à l'humidité qui comprend une pellicule ou une feuille de polymère perméable à l'humidité, un gel aquifère (3) qui comprend un polymère hygroscopique et de l'eau, et un matériau de recouvrement (4), lesquels sont laminés dans cet ordre, dans lequel le matériau de base (2) et le matériau de recouvrement (4) sont collés ou fondus ensemble à leurs périphéries, **caractérisé en ce que** la perméabilité à l'humidité du matériau de base et celle du matériau de recouvrement sont respectivement régulées dans la plage de 2500 à 4500 g/m².24 heures et de 200 g/m².24 heures ou moins selon le procédé de Lyssy.

2. Dispositif selon la revendication 1, dans lequel le matériau de base (2) comprend en outre un matériau de tissu fabriqué à partir d'une fibre naturelle et/ou artificielle sur la surface externe de la pellicule ou de la feuille de polymère perméable à l'humidité.

3. Dispositif selon la revendication 2, dans lequel le matériau de tissu est choisi dans le groupe constitué par un tissu, un tissu de serviette, un tissu d'une couverture, un tissu piqué, un tissu tricoté, un tissu non tissé, et un tissu tissé.

4. Dispositif selon la revendication 1, dans lequel la pellicule ou la feuille de polymère est fabriquée à partir d'au moins un membre choisi dans le groupe constitué par le polyuréthane, le polyéthylène, le copolymère d'acétate de vinyle et d'éthylène, le poly(chlorure de vinyle), le polyamide, le polyester, le polyacétal, la polysulfone, le poly(oxyde de phénylène), le polybutadiène, le polypropylène, la cellulose régénérée, le polychloroprène, un poly(acide aminé), le caoutchouc de nitrile, le caoutchouc de butyle, et le caoutchouc de silicone.

5. Dispositif selon la revendication 1 ou 2, dans lequel le matériau de base (2) comprend en outre, pour réguler la perméabilité à l'humidité du matériau de base (2), une pellicule ou une feuille (7) qui est fabriquée à partir d'au moins un membre choisi dans le groupe constitué par le polyuréthane, le polyéthylène, le copolymère d'acétate de vinyle et d'éthylène, le poly(chlorure de vinyle), le polyamide, le polyester, le polyacétal, la polysulfone, le poly(oxyde de phénylène), le polybutadiène, le polypropylène, la cellulose régénérée, le polychloroprène, un poly(acide aminé), le caoutchouc de nitrile, le caoutchouc de butyle, et le caoutchouc de silicone sur la surface externe de la pellicule ou feuille perméable à l'humidité ou du matériau de tissu.

6. Dispositif selon la revendication 5, lequel comprend en outre un dispositif (6) pour maintenir la pellicule ou la feuille (7) afin de réguler la perméabilité à l'humidité du matériau de base (2) sur la surface externe du matériau de base (2), dans lequel le dispositif (6) est choisi dans le groupe constitué par une poche, une fermeture Velcro (marque déposée), un bouton pression, et une ceinture.

7. Dispositif selon la revendication 1, lequel comprend en outre une couche (5) ayant une tendance à se coller sur le matériau de base (2) sur l'ensemble ou une partie de la périphérie du matériau de base (2).

8. Dispositif selon la revendication 7, dans lequel la surface efficace de la couche (5) ayant une tendance à se coller est variable.

9. Dispositif selon la revendication 1, dans lequel le matériau de base (2) supporte, retient, ou imprègne au moins un élément choisi dans le groupe constitué par un médicament absorbant percutané ou endermique, un produit de beauté, et un émetteur d'infrarouge lointain.

10. Dispositif selon la revendication 1, dans lequel le gel aquifère (3) supporte, retient, ou contient au moins un élément choisi dans le groupe constitué par un médicament absorbant percutanée ou endermique, un produit de beauté, et un émetteur d'infrarouge lointain.
